# EUROPEAN PATENT APPLICATION

(11) **EP 4 068 168 A1**
(43) Date of publication of application: **05.10.2022**
(21) Application number: 21465514.4
(22) Date of filing: 29.03.2021
(51) Int. Cl.: G06N 5/02, G06N 5/04, G06N 5/00

(54) **SYSTEM AND METHODS FOR KNOWLEDGE REPRESENTATION AND REASONING IN CLINICAL PROCEDURES**

(71) Applicant: Siemens Healthcare GmbH, 91052 Erlangen (DE)
(72) Inventor: ULLASKRISHNAN, Poikavila, Cranbury, NJ 08512 (US); PASSERINI, Tiziano, Plainsboro, NJ 08536 (US); SHARMA, Puneet, Princeton Junction, NJ 08550 (US); KLEIN, Paul, Princeton, NJ 08540 (US); LILIAC, Teodora Vanessa, 500239 Brasov (RO); MICU, Larisa, 500014 Brasov (RO)
(74) Representative: Schwarz und Baldus Patentanwälte

(57) **Abstract**

In one aspect the invention relates to a method for upgrading a medical knowledge base in a digital, clinical system, comprising the method steps of:
- Providing (S1) a storage with a knowledge base, being a SNOMED knowledge base, in a web ontology format;
- Receiving (S2) procedural data, representing clinical procedures for evaluation of a patient's health state;
- Mapping (S3) the received procedural data in a set of SNOMED expressions;
- Converting (S4) the SNOMED expressions into statements in the web ontology format;
- Upgrading (S5) the SNOMED knowledge base with the received procedural data by adding the statements in the SNOMED knowledge base for providing (S6) a processable file (PF) with an upgraded version of the SNOMED knowledge base.

## Description

The present invention relates to an apparatus, a product, a method and a system for representation of medical knowledge for digital data processing.

Medical scientific research is evolving fast. Therefore, medical knowledge needs to be updated regularly to keep track with new developments.

In this regard, implementations of clinical guidelines exist, which may be used in clinical decision systems. Usually, these implementations are rule-based as, for example, by using decision tree implementations.

Generally, a decision tree can be linearized into a set of decision rules, where the outcome is the content of the leaf node, and the conditions along the path form a conjunction in the if-clause. In general, the rules have the form:
"if condition1 and condition2 and condition3 then outcome".

Decision rules can be generated by constructing association rules with the target variable on the right. They can also denote temporal or causal relations.

However, these implementations of clinical guidelines have limitations, because typically the logical model and knowledge representation of different implementations "below" the application layer is inhomogeneous and may differ from system to system. Therefore, a global collaboration system cannot be achieved.

In state-of-the-art it is also known to use knowledge bases, using graph structured data models, like knowledge graphs. Knowledge graphs provide an alternative methodology to model rules for clinical decision support systems and there is much research that shows their advantage in resolving problems in the knowledge representation and automatic reasoning and decision support systems. Knowledge graphs may make use of intelligent machine learning algorithms and/or neural network technologies. Knowledge graphs may provide interfaces for associated software modules and systems. In particular, in the healthcare domain a standardized ontology exists, the SNOMED ontology. However, for being able to use this technology of knowledge graphs in healthcare, generally, it is required that knowledge representation systems and related automatic support systems are internationally valid and may be used in different countries for different local requirements and with different local settings, comprising medical questions and/or specializations.

Another requirement in the medical domain is, that often medical data (e.g., health-related measurements, vital data, anamnestic data) is missing which actually is necessary for providing a clinical decision. Further, not seldom decisions have to be taken on an urgent basis for initiating life-threatening measures. Also, in this respect it would be helpful to provide an understanding of the reasoning and internal decisions (for example which branches of a graph have been activated or which path in the graph has been taken), which have been taken by an automatic decision-support system, when providing a final result. With other words, it would be helpful to not only provide the final decision, but also to provide interim results so to say on the way to the final decision to get more transparency for the interference machine and into the decision logic.

Generally, decision tree-based implementations have drawbacks. First, it is not possible to reach a conclusion (typically represented in traversing the tree down to a leaf) if it is not possible to provide all required variables or data.

Second, it is difficult or even impossible to provide other information than modeled within the decision tree, once it has been generated.

Third, as decision trees are "hard-coded" it is difficult to update clinical knowledge and to provide machine learning capabilities.

Therefore, an object of the present invention is to provide improved mechanisms and tools for knowledge representation for medical knowledge, which might be used by automatic clinical decision support systems. Further, automatic machine interference should be made as transparent as possible and it should be possible to continuously update the knowledge base.

Further, it would be helpful to use the interference capabilities of knowledge graphs and also the inherent semantic relations of a certain ontology and not only to provide a clinical decision support system which points to an entry in the ontology data representation (e.g., SNOMED) as a result.

The above-mentioned object is solved with a method, apparatus, a system and the computer readable medium according to the attached independent claims. Further advantages embodiment, features and/or advantages are mentioned in the dependent claims.

In this respect it has to be mentioned, that the following description relates to the embodiment of the invention, represented in the method claim and its possible alternative embodiments. Features, advantageous embodiments and optional alternatives, which are claimed and/or described with reference to the method may also be used in and thus transferred to the other claim types, like for example to the apparatus or to the computer program product and vice versa. From a computer scientist's point of view, a hardware implementation is to be regarded as equivalent to a software implementation. Therefore, any features mentioned with regard to the method (for example "storing") may accordingly be transferred and applied to the hardware solution and will be implemented as hardware module with the respective functionality ("storage means" with a storing functionality). For avoiding redundancies, any advantageous embodiments or features which are mentioned with respect to the method are not reiterated again for the apparatus or product claims.

Further, it is to be pointed out that software typically is modular in nature. Thus, a specific implemented feature which is mentioned in combination with a certain embodiment may also be combined with other features, even when mentioned in other embodiments. Accordingly, any feature may be combined with at least one other feature, which is claimed and/or described in this application.

With this invention, it is possible to implement procedural data, for instance clinical knowledge, e.g., in the form of guidelines onto existing ontologies so that it is possible to generate a new and updated ontology.

In particular, each guideline is treated as a set of edges between existing nodes, representing a new pathway in a knowledge graph. SNOMED ontology is taken as the base ontology due to it being the most widely used and most complete clinical terminology.

For processing these ontologies, standard ontology languages, like a Web Ontology Language (OWL) may be used, which is a family of knowledge representation languages for authoring ontologies. Further, a Resource Description Framework (RDF) may be used.

A knowledge base may be structured as a knowledge graph. The knowledge graph may be subject to a decision-tree inference system. In machine learning and decision analysis, a decision tree can be used to visually and/or explicitly represent decisions and decision making. It may use a tree-like model of decisions. A decision tree is, thus, a tool for deriving a strategy to reach a particular decision. Decision Trees are a supervised learning method used for classification or regression tasks. The goal is to create a model that predicts e.g., a health-related decision by learning simple decision rules inferred from the data features and procedural data. A tree can be seen as a piecewise constant approximation.

The invention is based on SNOMED. SNOMED is a multilingual thesaurus with an ontological foundation. The use of SNOMED makes clinical information available in a computable form and thus processable. Thus, clinical or healthcare data can be queried and used to trigger decision support rules and prompts. The hierarchies of SNOMED enable complex reasoning to support decision support rules. For example, in SNOMED the concept |stroke| is synonymous with |cerebrovascular accident| and subsumes all lower-level concepts including |paralytic stroke|, |thrombotic stroke| etc. This means that decision support queries are easier to develop and implement because they do not need to identify all the individual terms and codes which may be relevant. Moreover, SNOMED provides tools to extract their ontology to standard OWL language which can be interpreted by any semantic web reasoner.

SNOMED also has the concept of Expressions wherein pathways within their ontology can be represented in the form of equations and their own compositional grammar structure. For more details it is referred to SNOMED CT Compositional Grammar (2020), retrievable from https://confluence.ihtsdotools.org/display/SLPG/SNOMED%2BCT%2B Compositional%2BGrammar.

In the invention, procedural data, for instance, in the form of a guideline may first be mapped - preferably manually - to a set of SNOMED expressions. Each leaf in the guideline has one SNOMED expression that equates it to the input variables in the guideline. This allows its mapping to SNOMED concepts and attributes. No new classes or attributes are developed for this. The SNOMED expressions are then converted to OWL statements (statements in the Web Ontology Language) that are appended to the released SNOMED OWL file. This creates a new upgraded SNOMED+ OWL file which can form the backbone for a clinical decision support system.

In a deployment scenario, patient data in an electronic healthcare record (EHR) is mapped to a set of SNOMED concepts. There are existing tools that already do this like SemRep [see, for more details: Kilicoglu, H., Rosemblat, G., Fiszman, M. et al. Broad-coverage biomedical relation extraction with SemRep. BMC Bioinformatics 21, 188 (2020). Retrievable from: https://doi.org/10.1186/s1285 9-020-3517-7]. Many hospitals also encode their data with SNOMED for standardization. An ontology reasoner can then use the guideline-enhanced SNOMED+ ontology provided by the tools of this application and infer if these patients belong to any specific classes. Ontology reasoners like e.g., Hermit [Hermit Reasoner (2013)] and compilers like Protégé [Protege OWL Environment (2011)] are well-established tools that are standard and can be used off-the-shelf. Descriptor Logic (DL Queries) can be used to also know direct and inferred super-classes that the patient belongs to.

According to a first aspect the present invention relates to a computer-implemented method for upgrading a medical knowledge base in a digital, clinical system, comprising the method steps of:
- Providing a storage with a knowledge base, being a SNOMED knowledge base, in web ontology format (e.g., OWL or RDF or XML statements);
- Receiving procedural data, representing clinical procedures for evaluation of a patient's health state (e.g., guideline data, in particular cardiological guideline data, like ECS data, ECS: European Society of Cardiology and/or clinical knowledge);
- Mapping the received procedural data in a set of SNOMED expressions;
- Converting the SNOMED expressions into statements in the web ontology format;
- Upgrading the SNOMED knowledge base with the received procedural data by adding the statements in the SNOMED knowledge base for providing a processable file with an upgraded version of the SNOMED knowledge base.

In a preferred embodiment, the knowledge base comprises or is based on a medical semantic network and in particular may apply a graph-based decision tree. For more technical details with respect to graph-based decision trees and its functionality it is referred to Corbett D.R. (2008) Graph-Based Representation and Reasoning for Ontologies. In: Fulcher J., Jain L.C. (eds) Computational Intelligence: A Compendium. Studies in Computational Intelligence, Vol. 115. Springer, Berlin, Heidelberg. https://doi.org/10.1007/978-3-540-78293-3_8.

The invention provides significant advantages.

First, it is possible to provide a result, for example, a decision with respect to a health state of a patient, even in the case data is missing or is not complete. This is possible because the invention is based on a graph-based decision tree. Due to the acyclic and non-directional nature of graphs as opposed to (normal) decision trees that can only move forward if all conditions of previous steps have been met, the graph-based decision tree according to the solution presented herein may also provide data in difficult scenarios, with e.g., missing or incomplete data.

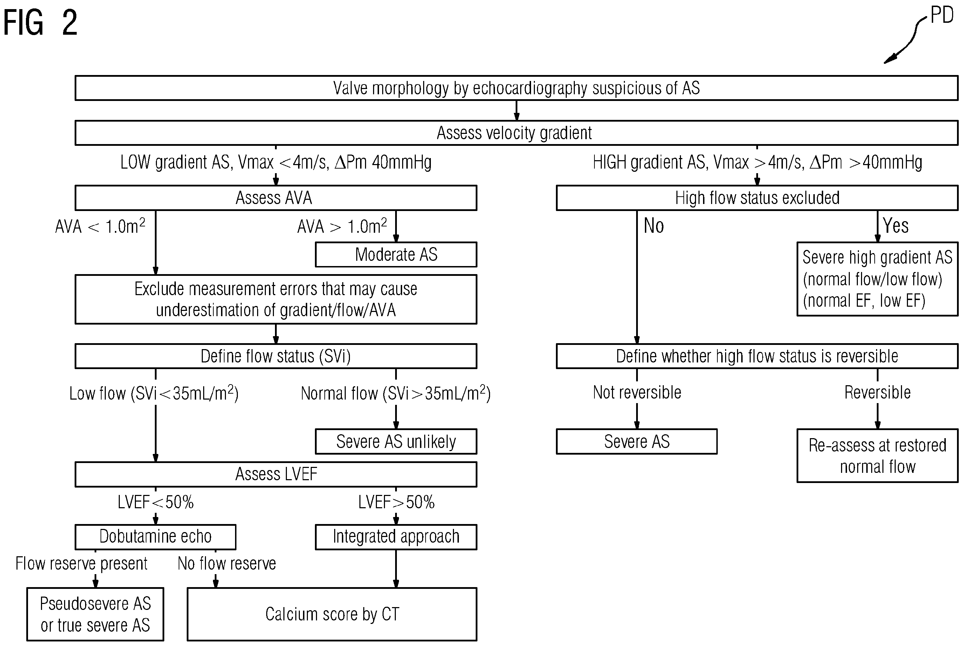

Second, the inference for the decision may be made transparent. In particular, upon user request, he or she is informed about automatic decision processing, i.e., which branches and paths in a graph-based decision tree have been activated. Since a graph is based on parent-child-attribute relationships, the end leaf node classification can be traced back to the uppermost parents in the graph of all variables that led to it. In this way, the trace provides a method for the user to know the path taken by the graph to arrive at the classification. It is more explainable. For more details it is referred to the Figures, in particular to the description of Fig. 2.

Third, it is very easy to upgrade the knowledge base and/or the rules for automatic decision execution. Since a graph is acyclical and non-directional, if a relation between two variables is established, the link can just be added. The entire decision tree doesn't have to be modified and tested. The ontology reasoner algorithm will function as usual and take into consideration the new linked entity in its reasoning. In this way, it is very easy to add/delete/modify relations without requiring a redesign of the entire decision tree. For example, if "hyperthyroidism" has to be added as a requirement for "moderate AS", the SNOMED concept for positive hyperthroidsm has to be just added as attribute to moderate AS.

With this invention specific types of inference are possible and processable which would not be possible with a normal decision tree. For example, the SNOMED concept for Aortic Stenosis may be linked to other SNOMED concepts regarding LVEF, AVA, Vmax and Delta PM (Pressure Gradient) through SNOMED relationships, wherein LVEF - Left Ventricle Ejection Fraction, AVA (Aortic Valve Area), Vmax - Maximal Flow Velocity in Valve, Delta PM (Pressure Gradient). In SNOMED ontology, since the SNOMED concept for Aortic Stenosis is defined by LVEF values as well, a SNOMED-based graph can detect automatically that the values are discordant and that the patient could be having "pseudosevere AS". Thus, clinical semantic knowledge embedded in SNOMED is used for more accurate classifications in this technology.

In another preferred embodiment, the knowledge base may be extended by further ontologies. For instance, ICD codes used for classification of diseases may also be used and applied in the form of an ontology, i.e., more detailed versions of a particular disease are children of the parent abstract disease. ICD codes are used in electronic health record / EHR systems to code the disease that a patient had and also for billing purposes. The ICD ontology can be linked to the SNOMED ontology in the same manner as concatenating two graphs. In such a scenario, the reasoning can be done not just based on SNOMED input variables but also on ICD input variables, thus enriching the pathway. Classification output can also be either SNOMED or ICD.

In another preferred embodiment, a specific patient instance may be applied to the upgraded processable file for inference of the patient's health state by means of loading the upgraded processable file in an ontology reader. In particular, the ontology reader may be configured to implement a reasoning algorithm. In particular, the reasoning algorithm may be or may comprise of classification algorithm in order to classify a patient's health state into disease categories and/or measure categories, representing medical measures to be performed.

In another preferred embodiment, the processable file is capable to provide result data even if a parameterization is incomplete, and/or parameters are inconsistent. For example, in patients with cardiac catheterization providing direct measurement of the pressure gradient across the valve or by alternative methodologies, like patient-based physiological modelling, measurement of blood velocity through Doppler signal processing may not be required to distinguish between low and high gradient AS. In a decision tree method, if the blood velocity Vmax is not present, it wouldn't function to provide a result, as that is the first entry point. But in a graph, if the AVA value is directly provided, it can skip the first requirement and continue. This is, because there is no precondition or direction in graph-based reasoning that blocks classification. If values can be provided within the pathway, the graph can continue from that point.

In still another preferred embodiment, the method may further comprise the step of:
- generating a virtual representation of the decision tree processing. The virtual representation may be stored and provides an automatically generated documentation of the inference and decision tree execution, so that the processing becomes more transparent and interim results may be documented as well. Alternatively, or in addition, the virtual representation may be used to automatically validate and, if needed, correct the processing. Moreover, by using the virtual representation, a secondary system may automatically validate and/or infer the processing to be carried out.

In another preferred embodiment, the knowledge base is augmented by numerical measurement values acquired by a set of medical devices. The medical devices may comprise a temperature sensor, a device for measuring blood composition and values and/or acquisition devices, in particular devices for acquiring physiological parameters or others.

In still another preferred embodiment, the knowledge base is augmented by medical image data acquired by a set of medical imaging devices, like CT, MRI, ultrasound, and/or others.

In still another preferred embodiment, the upgraded SNOMED knowledge base is used in a clinical decision support system (CDS) for providing a classification result dataset. The classification task is determined on a case-by-case basis and according to the respective setting. The classification may e.g., be in two or more different classes, like healthy and non-healthy.

In still another preferred embodiment, the classification result dataset comprises a prediction for a health-related measure and/or process and/or a patient's health state, a confidence range, further clinical measures and/or an inference dataset, representing a trace of an automated reasoning leading to the classification result dataset. The technical effect of this features is to enhance transparency of automatic inference.

Preferably, the method may comprise a machine learning algorithm. The machine learning algorithm may be configured to comprise instructions for processing input data to obtain output data and at least some of these instructions are set by using a set of training data and a training algorithm. The machine learning algorithm may be a supervised learning algorithm. It may be based on a convolutional neural network and/or a deep neural network, a decision tree, a random forest, a support vector machine (SVM), or other architectures.

Cumulatively or in addition, the machine learning algorithm may respectively use or comprise a decision tree that processes input data to "decide", e.g., provide an estimate, of a patient's health state, in particular providing an estimate of which kind of device-based measure, procedure or care (e.g., with an imaging device) is necessary for the patient.

In still another preferred embodiment, a trained graph-based convolutional neural network may be used for performing a data processing task, like a classification task with respect to the patient's health status. Alternatively, or in addition, a deep neural network (DNN) and in particular a trained graph-based convolutional neural network (CNN) may be used for estimating missing or incorrect data.

The training data of the neural network may be an instance of an incomplete data set (very different medical and/or healthcare data types and/or parameters are missing) and an instance of a correct and complete dataset, which is related and associated to the incomplete data set and/or manually checked. After training, the neural network is then used to automatically complement or complete insufficient and/or inconsistent and/or partial data sets.

In still another preferred embodiment, self-explanation techniques are applied for explaining convolutional neural network interference. Preferably, the self-explanation techniques may be based on a layer-wise relevance propagation technique, LRP).

Interpretability is especially important in applications such as medicine, where the reliance of the model on the correct features must be guaranteed. Therefore, transparency is important and has the technical effect that it is possible to provide an explanation of algorithmic (and thus automatic and "hidden") decisions. With this feature, it is possible to explain why a model is making the respective predictions. This serves to improve reliability.

An approach to explaining the prediction of the deep neural network (DNN) is to make explicit use of its graph structure, and proceed as follows: It is started at the output of the network. Then, it is moved in the graph in reverse direction, progressively mapping the prediction onto the lower layers. The procedure stops once the input of the network is reached. Layer-wise mappings can be engineered for specific properties.

Layer-wise relevance propagation (LRP), for example, is applicable to general network structures including DNNs and kernels. The layer-wise mappings are designed to ensure a relevance conservation property, where the share of received by each neuron is redistributed in same amount on its predecessors. The injection of negative relevance may be controlled by hyperparameters. For more details of the Layer-wise relevance propagation (LRP) it is referred to S. Bach, A. Binder, G. Montavon, F. Klauschen, K.-R. Müller, W. Samek, "On pixel-wise explanations for non-linear classifier decisions by layer-wise relevance propagation", PLoS ONE, 10 (7) (2015), Article e0130140.

In a preferred embodiment, a reasoner is used. The provided processable file may be forwarded and provided to the reasoner. The reasoner is configured to be sound, complete, and terminating: i.e., all entailments it finds do indeed hold, it finds all entailments that hold, and it always terminates. An "inference engine" may rely on a reasoning algorithm, implemented in a reasoner. The reasoner may be used for different purposes, like classification or other reasoning services. The invention makes use of automated reasoners such as Pellet, FaCT++, HerMiT, ELK etc. to take a collection of axioms written in OWL and offer a set of operations on the ontology's axioms.

In still another preferred embodiment, the method comprises accessing an extraction tool for extracting selected features of a SNOMED ontology.

The order, according to which the steps of the method of the present invention are described in the present specification, does not necessarily reflect the chronological order, according to which said steps are carried out.

According to another aspect, the invention refers to an apparatus for upgrading a medical knowledge base in a digital, clinical system, comprising:
- A first input interface to a storage for storing a knowledge base, being a SNOMED knowledge base, in web ontology format;
- A second input interface, configured to receive procedural data, representing clinical procedures for evaluation of a patient's health state;
- A processing unit for mapping the received procedural data in a set of SNOMED expressions;
- wherein the processing unit is further adapted for converting the SNOMED expressions into statements in the web ontology format;
- and wherein the processing unit is configured for upgrading the SNOMED knowledge base with the received procedural data by adding the statements in the SNOMED knowledge base for providing a processable file with an upgraded version of the SNOMED knowledge base.

In another aspect, the invention relates to a reasoning system, comprising:
- an apparatus, as mentioned above and
- the reasoner (interference engine).
The reasoning system may be or may be integrated in a clinical decision support system (CDS).

According to another aspect, the invention refers to a computer program product comprising program elements which induce a computer to carry out the steps of the method for upgrading a medical knowledge base in a digital, clinical system according to any of the preceding method claims, when the program elements are loaded into a memory of the computer.

According to another aspect, the invention refers to a computer-readable medium on which program elements are stored that can be read and executed by a computer in order to perform steps of the method for upgrading a medical knowledge base in a digital, clinical system according to any of the preceding method claims, when the program elements are executed by the computer.

The realization of the invention by a computer program product and/or a computer-readable medium has the advantage that already existing servers and/or clients can be easily adopted by software updates in order to work as proposed by the invention.

In the following a definition of terms used within this application is given.

A knowledge base is to be construed as a storage with a knowledge representation stored therein. A knowledge base may be a knowledge graph. A knowledge base serves as storage technology used to store and to make retrievable complex structured and unstructured information used by a computer system. An object model or ontology may serve as representation of knowledge. A knowledge base may be construed as a representation of heuristic and factual information, often in the form of facts, assertions and deduction rules. A knowledge base is usually used together with an inference engine (also called reasoner), which is a mechanism, playing the role of an interpreter, that applies the knowledge as represented in a suitable way to achieve results (for instance, automatic decisions, classification results etc.). Usually, a knowledge-based system further comprises interfaces for data exchange, in particular a user interface, with a mechanism that transfers queries from and answers to the user, sometimes seeking additional information for the Inference Engine. This includes explanation facilities for the user.

In particular, the knowledge base is a SNOMED knowledge base and is provided in a web ontology format. In a knowledge base, the knowledge representation should be a hybrid of many individual representations, such as frames, database facts, and deduction rules. Often, knowledge units expressed in one of these languages must be aggregated to the hybrid knowledge representation of one knowledge source which in turn must be further aggregated to a globally consistent knowledge base.

Within this application, the term "knowledge graph" is to be construed as that it acquires and integrates information into an ontology and applies a reasoner to derive new knowledge. For further definition it is referred to "Towards a Definition of Knowledge Graphs", L. Ehrlinger, W. Wöß, SEMANTICS 2016: Posters and Demos Track September 13-14, 2016, Leipzig, German. Thus, in this application a knowledge graph is somehow superior and more complex than a "normal" knowledge base (e.g., an ontology) because it is structured and formalized that it can be used by a reasoning engine (reasoner) to generate new knowledge and integrates one or more information sources.

SNOMED (or SNOMED CT, CT for clinical terms) is a systematically organized computer processable collection of medical terms providing codes, terms, synonyms and definitions used in clinical documentation and reporting. SNOMED is considered to be the most comprehensive, multilingual clinical healthcare terminology in the world. The primary purpose of SNOMED is to encode the meanings that are used in health information and to support the effective clinical recording of data with the aim of improving patient care. SNOMED provides the core general terminology for electronic health records. SNOMED comprehensive coverage includes: clinical findings, symptoms, diagnoses, procedures, body structures, organisms and other etiologies, substances, pharmaceuticals, devices and specimens. In this application SNOMED serves as a reference terminology in a multilingual form (i.e., enabling multilingual use) .

Generally, a storage may comprise volatile primary memory (e.g., a RAM, a DRAM, a SRAM, a CPU cache memory or the like) and/or non-volatile primary memory (e.g., a ROM, a PROM, an EPROM or the like). In particular, the volatile primary memory may consist of a RAM. For instance, the volatile primary memory temporarily holds program files for execution by the processing unit and related data and the non-volatile primary memory may contain bootstrap code for the operating system of the data processing system. The storage may further comprise a further memory, which may store the operating system and/or the instructions of the algorithms used to carry out the method of the present invention, in particular upgrading the SNOMED knowledge base. Moreover, the further memory may store a computer program product comprising instructions which, when the computer program product is executed by the processing unit, cause the processing unit to carry out the method according to the present invention.

Upgrading the knowledge base means to update the data storage with new data. This may preferably be done on a regular basis. This comprises to store additional data into the knowledge base in a related form, which means that new data is assigned and associated to respective corresponding entries in the knowledge base. So, new data is processable.

Procedural data is a set of digital data. Procedural data may be stored in a distributed manner at different storage locations. Procedural data represent clinical procedures for evaluation of a patient's health state or for measures to be taken in this respect, e.g., initiating medical examinations or evaluations (like image acquisition procedures etc.).

SNOMED expressions are portions in digital form which are processable in SNOMED. SNOMED expressions may be understood as a set of relationships between SNOMED Concept Codes. This basically is identical to an equation with SNOMED Concept codes as variables.

Statements in the web ontology format or language, in particular OWL statements, are statements to be processed digitally. The Web Ontology Language (OWL) is designed for use by applications that need to automatically process the content of information by algorithms and machines instead of just presenting information to humans. OWL facilitates greater machine interpretability of Web content than that supported by XML, RDF, and RDF Schema (RDF-S) by providing additional vocabulary along with a formal semantics. OWL has three increasingly-expressive sublanguages: OWL Lite, OWL DL, and OWL Full, which all can be used in this invention. OWL can be used to explicitly represent the meaning of terms in vocabularies and the relationships between those terms. This representation of terms and their interrelationships is called an ontology. Procedural data represent medical or clinical procedures or actions, like e.g., measurements with medical devices. Procedural data may comprise guideline data.

Wherever not already described explicitly, individual embodiments, or their individual aspects and features, described herein can be combined or exchanged with one another without limiting or widening the scope of the described invention, whenever such a combination or exchange is meaningful and in the sense of this invention.

The order, according to which the steps of the method of the present invention are described in the present specification, does not necessarily reflect the chronological order, according to which said steps are carried out.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality.

Advantages which are described with respect to a particular embodiment of present invention or with respect to a particular figure are, wherever applicable, also advantages of other embodiments of the present invention.

It shall be understood that a preferred embodiment of the present invention can also be any combination of the dependent claims or above embodiments or features with the respective independent claim(s).

These and other aspects of the invention will be apparent from and elucidated with reference to the embodiments described hereinafter.

### BRIEF DESCRIPTION OF THE DRAWINGS

Fig. 1 shows the architecture for implementing the method for upgrading a SNOMED knowledge base;
Fig. 2 is an example for a stepwise integrated approach for the assessment of Arctic stenosis security (left) and focus view (right side);
Fig. 3 is an example for a simplified guideline as procedural data;
Fig. 4 shows another example of trimmed SNOMED ontology;
Fig. 5 shows an example with three patient instances loaded onto a reasoner, working upon the upgraded knowledge base, being provided according to the invention;
Fig. 6 shows an example result, which has been classified by the reasoner to both moderate stenosis and the adult classes;
Fig. 7 depicts examples for direct and indirect SNOMED super-classes of a patient using descriptor logic query;
Fig. 8 is a flow chart of the method according to a preferred embodiment of the present invention;
Fig. 9 is another flow chart of optional procedures which may be applied on top of the general procedure represented in Fi. 8;
Fig. 10 is another flow chart of optional procedures which may be applied on top of the general procedure represented in Fig. 8;
Fig. 11 is a block diagram of an apparatus for executing the method according to a preferred embodiment of the present invention.

### DETAILED DESCRIPTION OF THE DRAWINGS

The present invention relates to a method and apparatus and related systems for upgrading a medical knowledge base with procedural data, like guideline data.

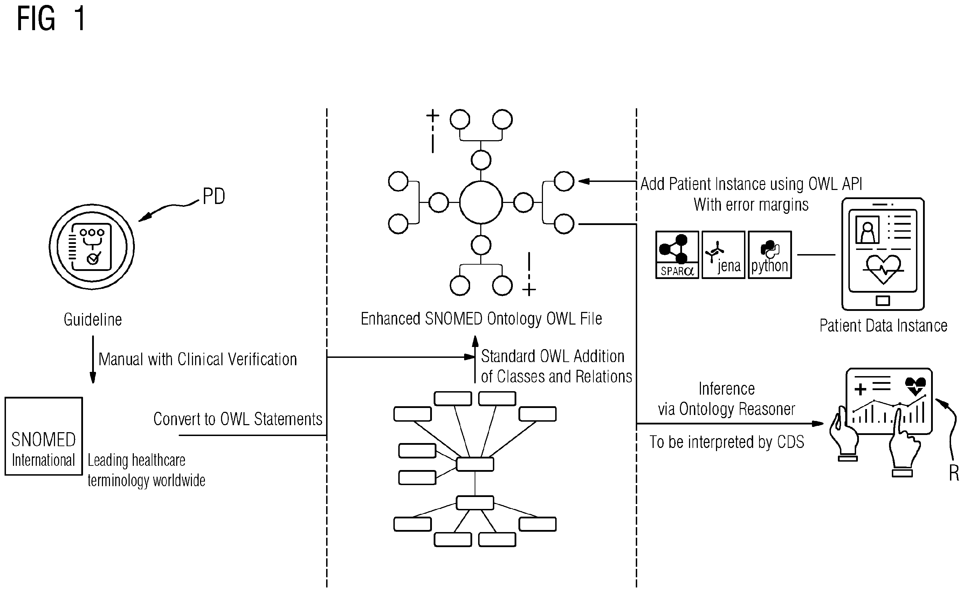

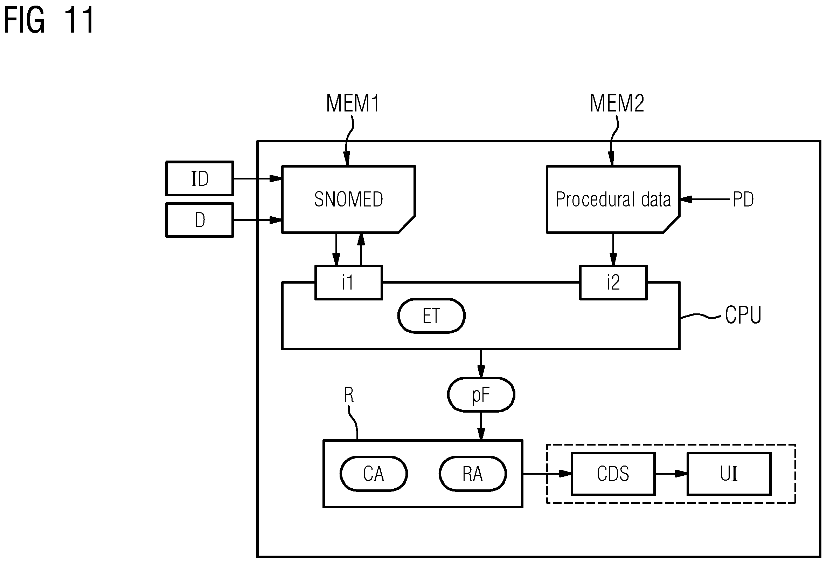

As can be seen in **Figure 1** procedural data PD should be applied to a knowledge base, being a SNOMED knowledge base.
The content of the knowledge base is converted to OWL statements, to provide an upgraded or enhanced SNOMED ontology OWL file which may be subject to further digital and automatic data processing. It is possible to add classes and relations and/or other entities according to the standard OWL language. Based upon the enhanced knowledge base, a specific patient instance may be added using an interface, for example an OWL API for inferring result data, by using an ontology reasoner R (see Fig. 11). The result may be interpreted by a clinical decision system CDS later on.

Without loss of generality, we consider for instance the translation of the clinical guidelines for the assessment of aortic valve stenosis into the upgraded knowledge base which has been provided with this invention and which may be denoted as SNOMED+ ontology.

An example is depicted in **Figure 2** as an example of a graphical representation of the recommended clinical decision process. As can be seen, the sequence of steps may be identified in the procedural data PD. For example, if the classification is "Moderate AS" (AS-Aortic Stenosis), the trace can read as follows:
"Moderate AS (Child)->AS(parent)->[AVA(attribute),Flow velocity(attribute),Pressure Gradient(attribute)]". This means that it was Moderate AS because of variables AVA, Flow Velocity and Pressure Gradient that affected its parent AS.
The trace will become more useful for much deeper graphs.

For example, the SNOMED concept for Aortic Stenosis is linked to other SNOMED concepts regarding LVEF, AVA, Vmax and Delta PM (Pressure Gradient) through SNOMED relationships, wherein LVEF - Left Ventricle Ejection Fraction, AVA (Aortic Valve Area), Vmax - Maximal Flow Velocity in Valve, Delta PM (Pressure Gradient). In the following scenario: Vmax and Delta PM are at thresholds and discordant or inconsistent to each other i.e., Vmax is < 4 and Pm > 40. In such a case a decision tree doesn't "know" which way to proceed. Even if the decision tree is programmed to take the left subtree, the AVA measurement could also be at threshold. If it is > 1, the decision tree will say "Moderate AS". Now, LVEF is a measurement independently taken from the image and is not derived from Vmax, AVA or Pm. Hence, the error is not propagated. If the LVEF value is much less than 50, a clinician will know that the patient could have "pseudosevere AS" and not "moderate AS". This could not be reflected in a usual decision tree as the LVEF value only comes into play down the tree and cannot be used to correct starting variables like AVA, Vmax or Delta PM. In SNOMED ontology, since the SNOMED concept for Aortic Stenosis is defined by LVEF values as well, a SNOMED-based graph can detect automatically that the values are discordant and that the patient could be having "pseudosevere AS". Thus, clinical semantic knowledge embedded in SNOMED is used for more accurate classifications in this technology.

The recommended decision process for the classification of the disease (here: aortic stenosis, AS) is based on the evaluation of multiple clinical statuses, which are combined into a final diagnosis. Each clinical status may be evaluated based on direct or indirect clinical measurements (e.g., measurement of maximal velocity Vmax through the aortic valve). SNOMED expressions can describe each path in the decision process through SNOMED concepts and attribute.

One advantage of this approach is that it allows to leverage equivalency between concepts through nested SNOMED expressions. For instance, 'high flow status' can itself be defined as an expression, to be evaluated based on a set of input variables (concepts and attributes). For the specific case of high flow status, conditions associated to it include anaemia, hyperthyroidism, presence of arterio-venous shunts. Assessment of high flow status can then be achieved in one or multiple ways, based on the same or different clinical guidelines, so that in the deployment scenario the decision pathway is automatically cleared based on available clinical information.

In the same spirit, a graph-based decision tree can also be augmented with decision pathways relying on additional information leading to the same diagnosis. For instance, in patients with cardiac catheterization providing direct measurement of the pressure gradient across the valve, measurement of blood velocity through Doppler signal processing may not be required to distinguish between low and high gradient AS (AS: aortic stenosis). Similarly, alternative decision processes can be designed based on alternative methodologies such as non-invasive estimation of blood pressure gradients based on patient-tailored physiological modeling. Such approaches may themselves be represented by SNOMED expressions, and rely on additional concepts and attributes (for instance, blood pressure can be estimated by physiological modeling based on 3D imaging and cuff blood pressure measurements). In this context, physiological modeling refers to estimation of physiological signals or phenomena based on clinical data, using various approaches including computational modeling or machine trained models. The reasoning system based on an upgraded SNOMED+ ontology described in this invention can be based on existing guidelines. Procedural data PD, for example, guideline data, can be extracted for instance from published clinical recommendations automatically. For this purpose, an extraction tool ET may be applied as a "crawler" for searching configured databases on a regular basis. Procedural data PD may also be based on clinical practice as defined within specific communities or healthcare systems (best practice, represented in a digital format). It can also be used to expand existing decision processes, for instance by defining specialized decision processes for subgroup of patients. One possible implementation of specialized decision processes is based on explicitly conditioning different SNOMED expressions based on patient characteristics or comorbidities. A simple example is adapting the thresholds in the decision process depicted in the picture above based on patient anatomical conditions (patients with small LVOT diameters, or generally patients of small stature are expected to have small valve area in presence of low pressure gradient in normal conditions). Another implementation is based on explicitly enriching the decision process by including non-imaging clinical observations (patient info, symptoms, clinical report of examination) and designing additional or alternative decision pathways based on those observations. One example of this approach is a decision process looking at patients for which diagnosis of aortic stenosis is difficult purely based on the standard measurements recommended by the guidelines - such as patients with stenotic valve in presence of low-pressure gradient, small aortic valve area and preserved ejection fraction. In this subgroup, collection of symptoms, personal data and additional measurements (e.g., calcium score) are all useful for the final determination.

One important application of the proposed system and method is that the system may serve as the backbone of interpretable decision support systems CDS.

The user of the system receives an estimated disease condition for a given patient, together with a trace of the reasoning leading to the decision. This can be augmented with confidence intervals which can be associated to each SNOMED expression, for instance as a function of the number of available input variable or the degree of certainty on each measurement.

The proposed system models the relationship between SNOMED concepts/attributes and the diagnosis through pre-defined SNOMED expressions. It can be expanded to create new expressions based on available concepts.

In a preferred embodiment, graph convolutional neural networks can be applied to model the relationship between SNOMED concepts and given disease labels, as following. An upgraded SNOMED+ ontology as being provided by the solution, presented herein, as processable file PF, can be represented as a graph, with SNOMED expressions encoding guidelines/decision processes being represented by sub- graphs. A training database is defined as a multitude of graphs, each corresponding to a subject (after mapping subject data to SNOMED+); each subject is associated to a disease label. Without loss of generality, we consider the example of aortic stenosis, for which the label can be: no disease, moderate disease, severe disease. Graph convolutional neural network process input data with a graph structure by computing predictive feature in each graph node based on information from the node neighbors. This allows such networks to be robust to changing graph architectures (corresponding to missing information) and to be sensitive to data patterns associated to nodes that are explicitly linked together. They are therefore suited for use on graphs defined based on ontologies such as SNOMED, in which node connections are based on semantic information based on medical knowledge. Finally, the neural network can be trained to perform a classification task by means of a classification algorithm CA, each class representing a disease condition.

Importantly, techniques to explain the behavior of the neural network (such as layer-wise relevance propagation) can be used to discover what SNOMED concepts or combination thereof are relevant for correct disease diagnosis in the given training population.

### Demo Use Case:

For demo purposes, the standard SNOMED ontology is augmented with a modified version of the above ESC guideline (ESC: European Society of Cariology) along with error margin incorporation.

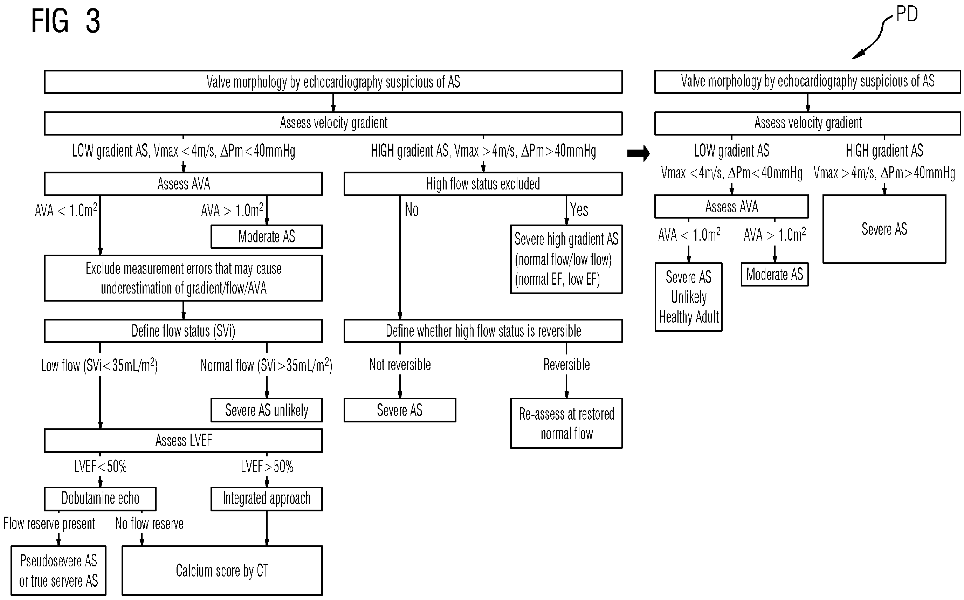

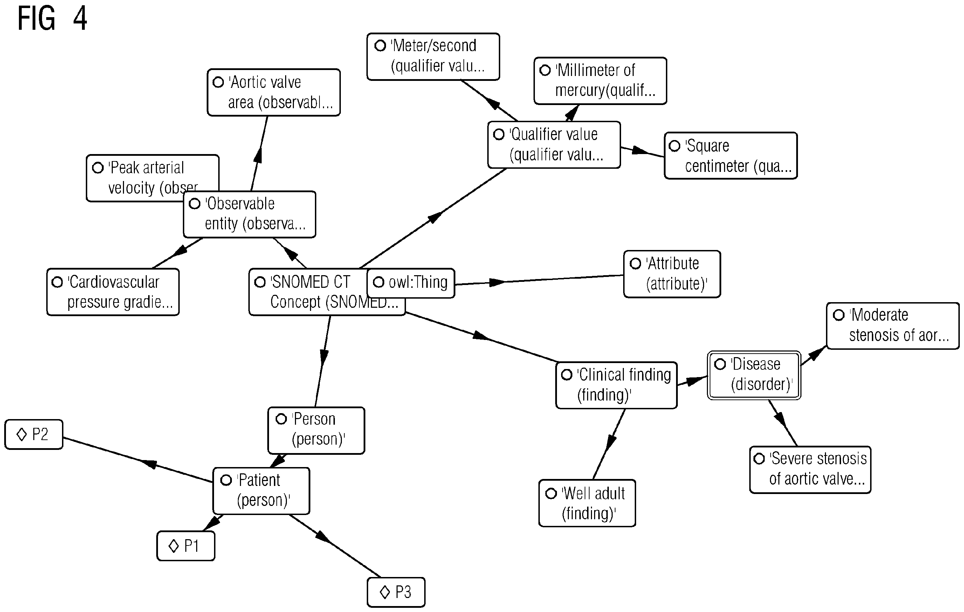

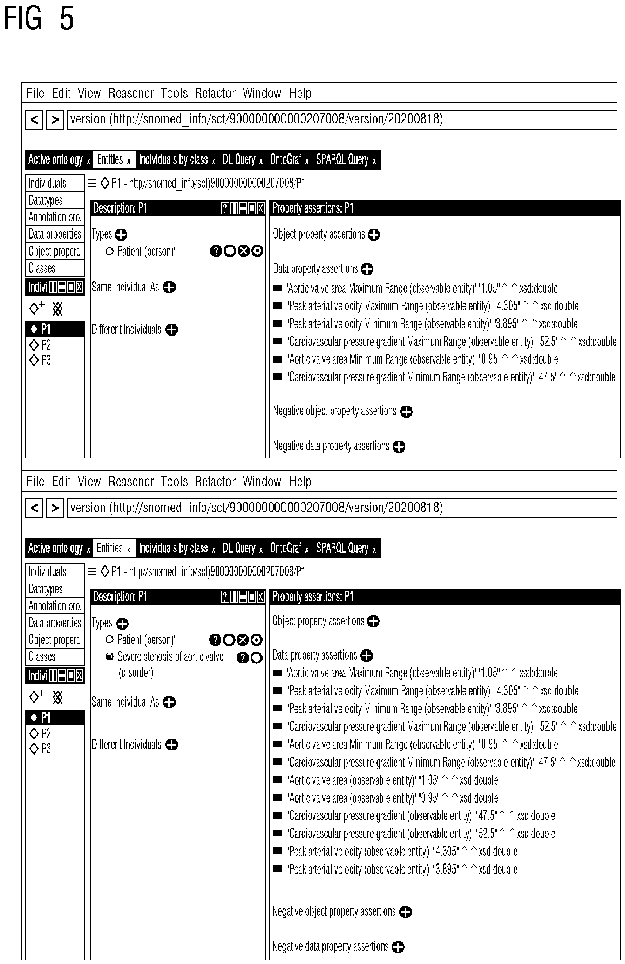

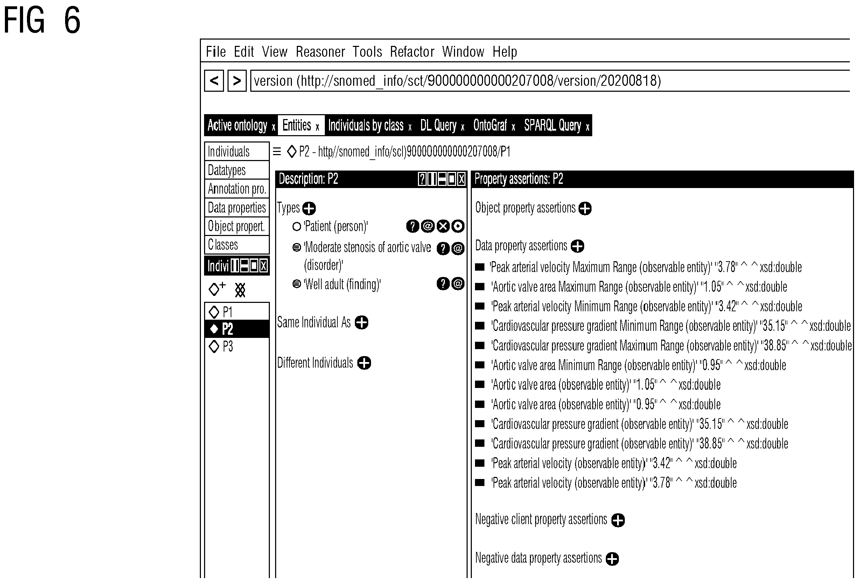

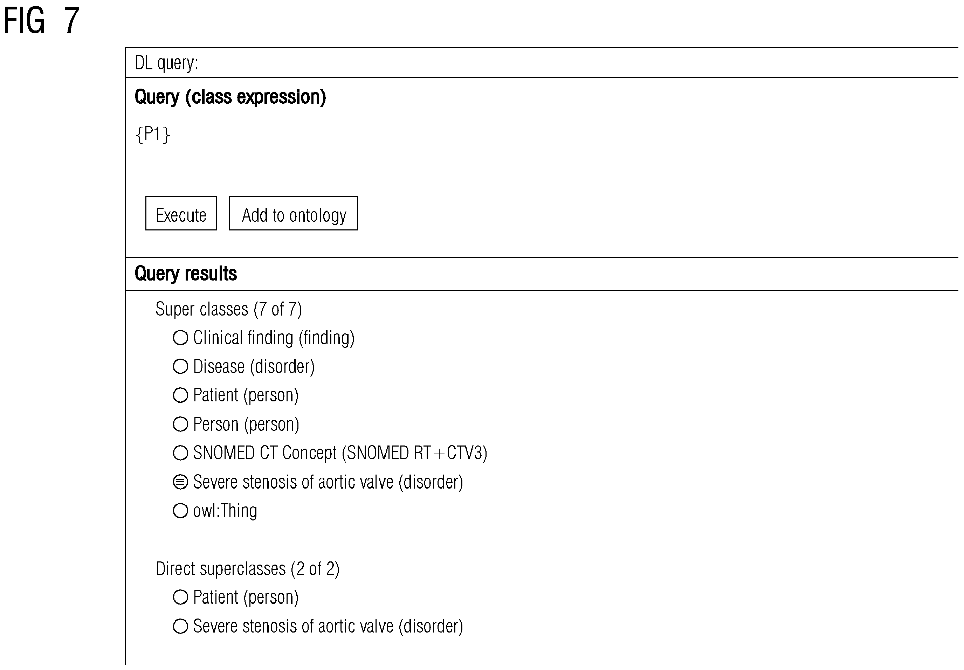

In the following steps which are undertaken, are listed:
1) The guideline is simplified even further for the purpose of demo and is shown in a graphical simplified schematic representation of **Figure 3****.**
2) The SNOMED ontology is also trimmed to only include concepts and relations that are relevant for this demo **(****Figure 4****)** .
3) The SNOMED ontology file is then converted to standard OWL format using the SNOMED OWL Toolkit, for example, SNOMED OWL Toolkit (2020). Retrieved from https://github.com/IHTSDO/snomed-owl-toolkit.
4) The simplified guideline is converted to the following SNOMED expressions so as to classify the three leaf nodes (Severe stenosis, Moderate Stenosis and No Stenosis or Healthy Adult). SNOMED compositional grammar is used to create the expressions and its description is beyond the scope of this document.
   836482000 | Severe stenosis of aortic valve (disorder) | === (252065006 | Peak arterial velocity (observable entity) |:732944001 | Has presentation strength numerator value (attribute) |>=#4+ 251081004 | Cardiovascular pressure gradient (observable entity) | :732944001 | Has presentation strength numerator value (attribute) |>=#40
   836481007 | Moderate stenosis of aortic valve (disorder) |===(252065006 | Peak arterial velocity (observable entity) | :732944001 | Has presentation strength numerator value (attribute) | <#4 + 251081004 | Cardiovascular pressure gradient (observable entity) | :732944001 | Has presentation strength numerator value (attribute) |<#40 + 251011009 | Aortic valve area (observable entity) | :732944001 | Has presentation strength numerator value (attribute) |>#1
   102512003 | Well adult (finding) | ===836481007 | Moderate stenosis of aortic valve (disorder) | ===(252065006 | Peak arterial velocity (observable entity) | :732944001 | Has presentation strength numerator value (attribute) | <#4 + 251081004 | Cardiovascular pressure gradient (observable entity) | :732944001 | Has presentation strength numerator value (attribute) | <#40 + 251011009 | Aortic valve area (observable entity) | :732944001 | Has presentation strength numerator value (attribute) | <=#1
5) The SNOMED expressions are then converted to a set of OWL statements and added to the trimmed SNOMED OWL file. Expressions can be written as equivalent classes in OWL RDF/XML syntax.
6) The input numerical variables are redefined to have maximum and minimum range values to account for the assumed 5% error rate.
7) 3 Patient instances are then added to the same OWL file to have values that lead to the three different classifications.
8) The processable file PF with the modified SNOMED+ OWL file can then be loaded onto any ontology reader R and then any standard reasoner can be applied. Protégé may be used for this purpose and the inbuilt Hermit reasoner may be used, too.
   As seen in **Figure 5****,** the reasoner R automatically classifies the patient instances to one of the three stenosis classes.
9) For patient P2 **(****Figure 6****),** the reasoner R (not shown n Fig. 6, however see Fig. 1 and 11) infers both Moderate Stenosis and Well Adult SNOMED classes since the Aortic Valve Area (AVA) was at the threshold of 1 cm2. Considering the error rate of 5%, P2 could be classified as having either of these conditions. Such a result of opposing classes can prompt the physician to confirm the value of AVA (aortic valve area) before further decisions.
10) One can also use the inherent SNOMED relations and standard ontology Description Logic queries to identify other classes to which the patient belongs **(****Figure 7****).** In this case, the reasoner R infers automatically that since P1 has Severe Stenosis, he/she should have a disease and thus also a clinical finding as super-classes. This is because in SNOMED, Sever Stenosis concept is classified as a disease and a disease is classified as a clinical finding. This can be extended to add other linked conditions, diseases or treatment options in the SNOMED ontology.

SNOMED's semantic network can be leveraged to make the guidelines better related to latest clinical knowledge. It also allows guidelines to be implemented without the need to re-define all possible concepts. SNOMED is not just used for reference literature but its semantic network, relations and concepts are used in addition to the guideline or other procedural data in the same upgraded ontology.

In this invention, an existing ontology is used as the base for adding guidelines. Standard ontology languages are used to facilitate easy plug-and-play of SNOMED versions and interpretation by various frameworks like Python/Java/Protege.

Other terminologies, like ICD-10, CPT, LOINC, can be linked to the ontology too using existing released mappings.

SNOMED expressions are leveraged to map to existing concepts and provide a method for cross-validation by multiple clinicians.

A graph-based decision tree also means that not all input variables are required to make decisions and it doesn't get blocked at any level. Definitions of relations between variables, either via SNOMED ontology or user-defined, can be used to assume values for missing variables. Confidence measures can be implemented to give probabilistic decisions depending on availability of data points.

As shown above, ontology-based reasoning can also show smarter decisions in threshold cases as opposed to deterministic outputs.

Changes in clinical knowledge can be updated either via new releases of SNOMED or adding them as new relations or concepts manually in the SNOMED OWL. A re-definition of the rules-engine is not needed, thus saving time and cost.

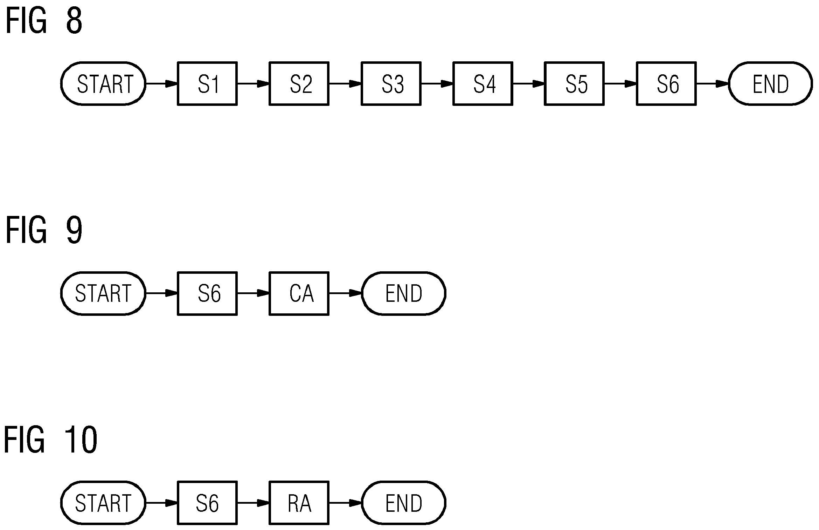

**Figure 8** shows a flowchart of a method for upgrading and/or operating a medical knowledge base. After start of the method, in step S1, a storage is provided with a knowledge base, being a SNOMED knowledge base, in a web ontology format (OWL). In step S2 procedural data, representing clinical procedures for evaluation of the patient's health state, like guideline data, are received. In step S3 the received procedural data is mapped to a set of SNOMED expressions. In step S4 the SNOMED expressions are converted into statements in the that ontology format. In step S5, the SNOMED knowledge base is upgraded with the received procedural data. This is preferably executed by adding the statements in the SNOMED knowledge base for providing processable file PF in step S6 with an upgraded version of the SNOMED knowledge base.

As can be seen in **Figure 9****,** further algorithms may be applied upon the upgraded version of the SNOMED knowledge base in the processable file PF. For example, after having provided the upgraded version of the SNOMED knowledge base in step S6, a classification algorithm CA may be applied in order to classify a patient's health state (for example in healthy or disease with different severities). After having provided the classification result, the method may end.

Another example of the application of a subsequent algorithm is shown in **Figure 10****.** After having provided the upgraded version of the SNOMED knowledge base in the processable file PF in step S6, a reasoning algorithm RA, implemented in the reasoner R, may be applied in order to show and make transparent the interference which has been used to come to the result, in particular to the classification result. The result is provided in a processable file, too. The processable file may be back propagated to the reasoner R.

**Figure 11** shows the architecture of an automatic reasoning system with an apparatus, which is configured to execute the method for upgrading a medical knowledge base. The apparatus comprises or is in data exchange with a first memory depicted in figure 11 with reference MEM1, in which the knowledge base, in particular the SNOMED knowledge base is stored. A second memory MEM2 is provided for storing guideline data or other procedural data PD. Depending on the specific embodiment, it is possible that the first and the second memory are implemented on the same or a different entities or units. The processing unit CPU comprises a first interface i1 for providing data exchange to the SNOMED knowledge base, in a web ontology format. A second input interface i2 is configured to receive procedural data PD, representing clinical procedures for evaluation of a patient's health state, e.g., guideline data. The processing unit CPU is configured for mapping the received procedural data in a set of SNOMED expressions. The processing unit CPU is further adapted for converting the SNOMED expressions into statements in the web ontology format and for upgrading the SNOMED knowledge base with the received procedural data by adding the statements in the SNOMED knowledge base for providing a processable file PF with an upgraded version of the SNOMED knowledge base. This can be seen in figure 11, the apparatus is shown in the middle part and mainly comprises the two interfaces i1, i2 and the CPU for data processing, in particular for executing the machine learning algorithms for providing the processable file PF.

The processable file PF is provided as output of the method and/or apparatus. The processable file PF may be processed by a reasoner R, which acts as inference engine on the processable file PF.

In a first embodiment, the reasoning system may comprise the apparatus and the reasoner R. The reasoner R may interact with an external clinical decision support system CDS, which may comprise a user interface UI for user interaction. This embodiment is shown in Fig. 11 with dotted lines.

In a second embodiment, the reasoning system itself is part of the clinical decision support system CDS. This is reflected in Fig. 11 with dashed lines.

As can be seen in Figure 11, the processing unit CPU may comprise an extraction to ET for extracting selected features of a SNOMED ontology. This feature improves performance of the method and may focus the upgrading process upon only relevant data. With this feature, execution time for the upgrading process may be reduced.

Any reference signs in the claims should not be construed as limiting the scope.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

## Claims

1. Computer-implemented method for upgrading a medical knowledge base in a digital, clinical system, comprising the method steps of:
- Providing (S1) a storage with a knowledge base, being a SNOMED knowledge base, in a web ontology format;
- Receiving (S2) procedural data, representing clinical procedures for evaluation of a patient's health state;
- Mapping (S3) the received procedural data in a set of SNOMED expressions;
- Converting (S4) the SNOMED expressions into statements in the web ontology format;
- Upgrading (S5) the SNOMED knowledge base with the received procedural data by adding the statements in the SNOMED knowledge base for providing (S6) a processable file (PF) with an upgraded version of the SNOMED knowledge base.

2. Method according to claim 1, wherein the knowledge base comprises a medical semantic network and in particular a graph-based decision tree.

3. Method according to any of the preceding claims, wherein the knowledge base is extended by further ontologies.

4. Method according to any of the preceding claims, wherein the upgraded SNOMED knowledge base is used for classification of a patient's health state by means of loading the provided processable file (PF) in an ontology reader, wherein the ontology reader may be configured for applying a classification algorithm (CA).

5. Method according to any of the preceding claims, wherein a specific patient instance may be applied to the processable file (PF) for inference of the patient's health state by means of an ontology reader for applying a reasoning algorithm (RA).

6. Method according to any of the preceding claims, wherein the processable file (PF) is able to provide result data even if a parameterization is incomplete, and/or parameters to be processed are inconsistent.

7. Method according to any of the preceding claims, wherein the knowledge base is augmented by numerical measurement values acquired by a set of medical devices (D).

8. Method according to any of the preceding claims, wherein the knowledge base is augmented by medical image data acquired by a set of medical imaging devices (ID).

9. Method according to any of the preceding claims, wherein the upgraded SNOMED knowledge base is used in a clinical decision support system (CDS) for providing a classification result dataset.

10. Method according to the directly preceding claim, wherein the classification result dataset comprises a prediction for a patient's health state, a confidence range, further clinical measures and/or an inference dataset, representing a trace of an automated reasoning leading to the classification result dataset.

11. Method according to the directly preceding claim, wherein self-explanation techniques are applied for explaining convolutional neural network inference.

12. Method according to any of the preceding claims, wherein the method comprises accessing an extraction tool (ET) for extracting selected features of a SNOMED ontology.

13. Apparatus for upgrading a medical knowledge base in a digital, clinical system, which is configured to perform the method according to any of the preceding method claims, comprising:
- A first input interface (i1) for providing (S1) a knowledge base, being a SNOMED knowledge base, in web ontology format;
- A second input interface (i2), configured to receive procedural data, representing clinical procedures for evaluation of a patient's health state;
- A processing unit (CPU) for mapping the received procedural data in a set of SNOMED expressions;
- wherein the processing unit (CPU) is further adapted for converting the SNOMED expressions into statements in the web ontology format;
- and wherein the processing unit (CPU) is configured for upgrading the SNOMED knowledge base with the received procedural data by adding the statements in the SNOMED knowledge base for providing a processable file (PF) with an upgraded version of the SNOMED knowledge base.

14. Automatic reasoning system for a clinical decision support system (CDS), comprising:
- An apparatus according to the directly preceding apparatus claim for upgrading a medical knowledge base and
- A reasoner (R) serving as an inference engine.

15. A computer-readable medium on which program elements are stored that can be read and executed by a computer to perform steps of the method for upgrading a medical knowledge base in a digital, clinical system according to any of the preceding method claims, when the program elements are executed by the computer.

## Amended claims

### Amended claims in accordance with Rule 137(2) EPC.

1. Computer-implemented method for upgrading a medical knowledge base in a digital, clinical system, comprising the method steps of:
- Providing (S1) a storage with a knowledge base, being a SNOMED knowledge base, in a web ontology format;
- Receiving (S2) procedural data, representing clinical procedures for evaluation of a patient's health state;
- Mapping (S3) the received procedural data in a set of SNOMED expressions;
- Converting (S4) the SNOMED expressions into statements in the web ontology format;
- Upgrading (S5) the SNOMED knowledge base with the received procedural data by adding the statements in the SNOMED knowledge base for providing (S6) a processable file (PF) with an upgraded version of the SNOMED knowledge base;
wherein the knowledge base comprises a medical semantic network, namely a graph-based decision tree;
and wherein the method further comprises:
- generating a virtual representation of the graph-based decision tree processing.

2. Method according to any of the preceding claims, wherein the knowledge base is extended by further ontologies.

3. Method according to any of the preceding claims, wherein the upgraded SNOMED knowledge base is used for classification of a patient's health state by means of loading the provided processable file (PF) in an ontology reader, wherein the ontology reader may be configured for applying a classification algorithm (CA).

4. Method according to any of the preceding claims, wherein a specific patient instance may be applied to the processable file (PF) for inference of the patient's health state by means of an ontology reader for applying a reasoning algorithm (RA).

5. Method according to any of the preceding claims, wherein the processable file (PF), if processed by the reasoner, is able to provide result data even if a parameterization is incomplete, and/or parameters to be processed are inconsistent.

6. Method according to any of the preceding claims, wherein the knowledge base is augmented by numerical measurement values acquired by a set of medical devices (D).

7. Method according to any of the preceding claims, wherein the knowledge base is augmented by medical image data acquired by a set of medical imaging devices (ID).

8. Method according to any of the preceding claims, wherein the upgraded SNOMED knowledge base is used in a clinical decision support system (CDS) for providing a classification result dataset.

9. Method according to the directly preceding claim, wherein the classification result dataset comprises a prediction for a patient's health state, a confidence range, further clinical measures and/or an inference dataset, representing a trace of an automated reasoning leading to the classification result dataset.

10. Method according to the directly preceding claim, wherein self-explanation techniques based on a layer-wise relevance propagation technique, LRP, are applied for explaining convolutional neural network inference.

11. Method according to any of the preceding claims, wherein the method comprises accessing an extraction tool (ET) for extracting selected features of a SNOMED ontology.

12. Apparatus for upgrading a medical knowledge base in a digital, clinical system, which is configured to perform the method according to any of the preceding method claims, comprising:
- A first input interface (i1) for providing (S1) a knowledge base, being a SNOMED knowledge base, in web ontology format;
- A second input interface (i2), configured to receive procedural data, representing clinical procedures for evaluation of a patient's health state;
- A processing unit (CPU) for mapping the received procedural data in a set of SNOMED expressions;
- wherein the processing unit (CPU) is further adapted for converting the SNOMED expressions into statements in the web ontology format;
- and wherein the processing unit (CPU) is configured for upgrading the SNOMED knowledge base with the received procedural data by adding the statements in the SNOMED knowledge base for providing a processable file (PF) with an upgraded version of the SNOMED knowledge base.

13. Automatic reasoning system for a clinical decision support system (CDS), comprising:
- An apparatus according to the directly preceding apparatus claim for upgrading a medical knowledge base and
- A reasoner (R) serving as an inference engine.

14. A computer-readable medium on which program elements are stored that can be read and executed by a computer to perform steps of the method for upgrading a medical knowledge base in a digital, clinical system according to any of the preceding method claims, when the program elements are executed by the computer.
